# EUROPEAN PATENT SPECIFICATION

(11) **EP 2 856 149 B1**
(45) Date of publication and mention of the grant of the patent: **08.07.2020**
(21) Application number: 13794110.0
(22) Date of filing: 24.05.2013
(51) Int. Cl.: G01N 33/48, G01N 33/564

(54) **DIABETES BIOMARKERS**
BIOMARKER FÜR DIABETES
BIOMARQUEURS DU DIABÈTE

(30) Priority: 24.05.2012 US 201261651144 P
(43) Date of publication of application: 08.04.2015
(73) Proprietor: DMNoMore, London House Suite 19 London SW10 9EL (GB)
(72) Inventor: ORBAN, Tihamer, Brookline, MA 02446 (US)
(74) Representative: Dehns
(86) International application number: PCT/US2013/042627
(87) International publication number: WO 2013/177505

(56) References cited:
- WO-A2-2009/120341
- MEHLING M ET AL: "FTY720 therapy exerts differential effects on T cell subsets in multiple sclerosis", NEUROLOGY, LIPPINCOTT WILLIAMS & WILKINS, PHILADELPHIA, US, vol. 71, no. 16, 1 October 2008 (2008-10-01), pages 1261-1267, XP009165260, ISSN: 0028-3878, DOI: 10.1212/01.WNL.0000327609.57688.EA
- XIAOFANG ZHANG ET AL: "Acute Response of Peripheral Blood Cell to Autologous Hematopoietic Stem Cell Transplantation in Type 1 Diabetic Patient", PLOS ONE, vol. 7, no. 2, 22 February 2012 (2012-02-22), page e31887, XP055265186, DOI: 10.1371/journal.pone.0031887
- ELENA MATTEUCCI ET AL: "Altered Proportions of NaÃve, Central Memory and Terminally Differentiated Central Memory Subsets among CD4and CD8T Cells Expressing CD26 in Patients with Type 1 Diabetes", JOURNAL OF CLINICAL IMMUNOLOGY, KLUWER ACADEMIC PUBLISHERS-PLENUM PUBLISHERS, NE, vol. 31, no. 6, 2 September 2011 (2011-09-02), pages 977-984, XP019983572, ISSN: 1573-2592, DOI: 10.1007/S10875-011-9573-Z
- TIHAMER ORBAN ET AL: "Co-stimulation modulation with abatacept in patients with recent-onset type 1 diabetes: a randomised, double-blind, placebo-controlled trial", THE LANCET, vol. 378, no. 9789, 1 July 2011 (2011-07-01), pages 412-419, XP055242611, GB ISSN: 0140-6736, DOI: 10.1016/S0140-6736(11)60886-6
- T. ORBAN ET AL: "Reduction in CD4 Central Memory T-Cell Subset in Costimulation Modulator Abatacept-Treated Patients With Recent-Onset Type 1 Diabetes Is Associated With Slower C-Peptide Decline", DIABETES, vol. 63, no. 10, 16 May 2014 (2014-05-16), pages 3449-3457, XP055266767, US ISSN: 0012-1797, DOI: 10.2337/db14-0047
- ADAM KRETOWSKI ET AL.: 'Ocena subpopulacji limfocyt6w T pomocniczych: naiwnych (CD4+CD45RA+), pamieci (CD4+CD45RO+) oraz wykazujacych ekspresje fenotyp6w CD45RA+ i CD45RO+ w przedklinicznej fazie cukrzycy typu 1 (prediabetes).' PRZEGLAG LEKARSKI vol. 58, no. 1, 2001, pages 16 - 19, XP008175726
- BART O. ROEP.: 'The role of T-cells in the pathogenesis of Type 1 diabetes: From cause to cure' DIABETOLOGIA vol. 46, no. 3, 2003, pages 305 - 321, XP055179757

## Description

### CROSS REFERENCE TO RELATED APPLICATIONS

The present application claims priority to U.S. provisional application Serial Number 61/651,144 filed May 24, 2012, herein incorporated by reference in its entirety.

### FIELD OF THE INVENTION

The present invention relates generally to the field of autoimmunity, diabetes and more particularly to Type 1 diabetes and immune markers.

### BACKGROUND

The most common form of Type 1 diabetes mellitus (T1DM) is an immune-mediated disease where insulin-secreting β-cells are destroyed by an autoimmune response. There are a number of genetic and environmental factors associated with the onset of the disease, which involves the progressive inflammatory infiltration of pancreatic islets by immunocytes targeted specifically to insulin-secreting β-cells. This pathology develops over an indeterminate period of time (months to years) prior the clinical onset (pre-diabetes) and continues after the patient is diagnosed with the disease.

There is currently a method for screening and diagnosis of T1DM using antibodies. Where the specific antibodies are present, over time, overt diabetes will often develop. Expression of one or more of: GAD65 autoantibodies (GAAs), ICA512 autoantibodies (ICA512AAs), or anti-insulin autoantibodies (IAAs) is associated with a risk of progression to T1DM. Expression of two or more of: GAD65 autoantibodies (GAAs), ICA512 autoantibodies (ICA512AAs), or anti-insulin autoantibodies (IAAs) is associated with a high risk of progression to T1DM. (Liping Yu et al., Diabetes August 2001 vol. 50 no. 8 1735-1740; Verge CF et al., Diabetes 45:926-933, 199; Verge CF. et al, Diabetes 47:1857-1866, 1998; *and* Bingley PJ, et al., Diabetes 43:1304-1310, 1994*).*

However, this screening has limited usefulness for the individual patient. While the antibody screen can detect a heightened level of risk of T1DM, the risk is based on the population in general and cannot inform the individual patient as to whether the disease will, for example, onset within the next several months or if the individual will likely be free of diabetes for the next 5- 10 years. The intensity of the autoimmune destruction varies patient to patient. Thus, there is a need for a diagnostic method that will inform an individual of personal risk of developing T1DM and can indicate the time frame of disease onset.

There is also need for improved primary endpoints for analysis of therapies for T1DM autoimmunity. For a therapy to receive regulatory approval from the FDA, clinical trials must show statistically significant effect at an appropriate primary endpoint. This treatment effect preferably demonstrates some obvious and clinically significant benefit. In case of treatment to prevent T1DM, the delay or absent of development of the clinical disease (this makes these trials 5 or 10 years long). In case of intervention in patients with clinical disease, the appropriate primary endpoints are metabolic control and diabetes complication related. Most commonly applied metabolic primary endpoint - short of the cure- is measurement of self-insulin production (stimulated C-peptide as a surrogate marker for preservation of β-cell function), others are HbAlc and insulin use. Improved β-Cell function in T1DM patient can predict better short and long-term clinical outcome can take several years to assess. For treatment of T1DM and complications, blood sugar levels can be monitored directly and improved glycemic control can be monitored through the levels of glycosylated hemoglobin (e.g. HbA_{1c}), which has been shown to be directly related to the risk of short and long term diabetic complications.

For therapies intended to preserve β-cell function in a post clinical phase of the disease, stimulated C-peptide concentration has been used to measure progression of T1DM (Palmer JP, et al., Diabetes 2004; 53:250-264). However, the use of C-peptide concentration requires repeated invasive testing (so called Mixed Meal Tolerance Test) over extended period of time, thus the trials last for a long time to allow for assessment of progression of the disease that reduces β-cell function and thus C-peptide concentration.

Therefore, there is a need for better markers to serve as primary endpoints for trials (both prevention and intervention trials) as well as better markers that can more rapidly and reliably indicate the progressiveness of T1DM autoimmunity, thus stage the prediabetic and diabetic condition. There is also a need for better assays that can measure the effectiveness of therapies for the treatment of T1DM and/or detect complications.

### SUMMARY

A new marker for self-insulin production decline in Type 1 diabetes has been found in the ratio the CD4 naive (CD45RO-CD62L+) to central memory (CD45RO+CD62L+) T-cell subpopulations and in the central memory (CD45RO+CD62L+) T-cell subpopulation levels. The present invention provides a method of determining the effectiveness of a therapy to preserve β cell function in a subject who has Type 1 diabetes mellitus comprising:
selecting a subject who has Type 1 diabetes mellitus undergoing said therapy, and
(i) measuring the CD4 central memory (CD45RO+CD62L+) T-cell subpopulation in a sample from said subject, and evaluating the effectiveness of the therapy, wherein a low or decreasing CD4 central memory T-cell level during said therapy indicates effective therapy; or
(ii) measuring the CD4 central memory (CD45RO+CD62L+) T-cell subpopulation and measuring the CD4 T-cell naive (CD45RO-CD62L+) subpopulation in a sample from said subject, and evaluating the effectiveness of the therapy, wherein a high or increasing ratio of the CD4 T-cell naive to CD4 central memory T-cell subpopulation during said therapy indicates effective therapy.

Preferably, the method further comprises determining the presence of a diabetes-related autoantibody.

In prediabetes setting, the presence of T1DM specific autoantibody indicates the presence of T1DM autoimmunity itself. Thus, in one aspect of the present invention, the method as described herein is combined with determining whether diabetes autoantibodies are present.

Preferably, the sample is incubated with a labeled antiCD45RO antibody and a labeled antiCD62L antibody prior to the measuring step.

Preferably, measuring comprises subjecting said sample to flow cytometry.

Preferably, said sample is a blood sample.

Preferably, the decreasing CD4 central memory T-cell level or the increasing ratio is relative to the level or ratio from said extracted sample at different time points.

Preferably, the decreasing CD4 central memory T-cell level or the increasing ratio is relative to a standardized level or ratio, or to level or ratio obtained from a sample extracted before therapy starts.

Preferably, a low or decreasing CD4 central memory T-cell level during said therapy indicates effective therapy.

Preferably, measuring comprises measuring both the CD4 central memory T-cell subpopulation and the CD4 T-cell naive subpopulation and wherein a high or increasing ratio of the CD4 T-cell naive to CD4 central memory T-cell subpopulation during said therapy indicates effective therapy.

These and other features of the embodiments as will be apparent are set forth and described herein.

### BRIEF DESCRIPTION OF THE DRAWINGS

The following drawings form part of the present specification and are included to further demonstrate certain aspects of the invention. The invention may be better understood by reference to one or more of these drawings in combination with the detailed description of specific embodiments presented herein.

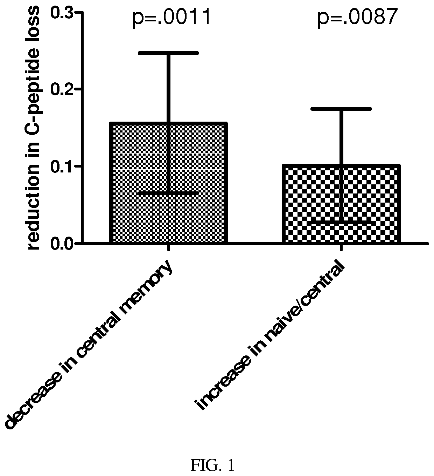

FIG. 1 is a chart showing the reduction in C-peptide loss per unit change in T cells at a prior visit compared to baseline. Both the decrease in central memory and the increase in naïve/central ratio are shown.

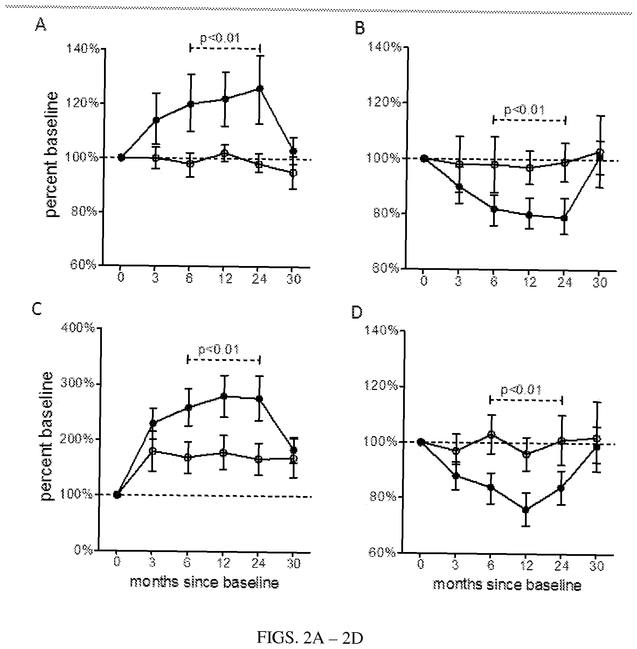

FIGS. 2A - 2D show the percent change from baseline of CD4 T cell subsets identified as representing (FIG. 2A) naive and (FIG. 2B) central memory populations as well as (FIG. 2C) the ratio of naive:central memory and (FIG. 2D) Treg populations, all measured at specified intervals after treatment initiation ("0 months"). Last treatment was at month 24. Closed circles are abatacept treated and open circles placebo; symbols represent mean and the error bars represent 95% confidence intervals. P values and dashed lines indicate that the two groups differ significantly over the timepoints indicated.

### DETAILED DESCRIPTION

The most common form of Type 1 diabetes mellitus (T1DM) is an immune-mediated disease where insulin-secreting β-cells are destroyed by an autoimmune response. T-cells play a central part in autoimmunity associated with TIDM. To become fully activated, these cells are believed to need at least two crucial signals. (Marelli-Berg FM, Okkenhaug K, Mirenda V. A Trends Immunol 2007; 28: 267-73.) The first signal is an interaction between an antigen in the groove of the MHC molecule on antigen-presenting cells with the T-cell receptor. The second signal is the interaction between CD80 and CD86 on the antigen presenting cells and CD28 on the T-cells. This co-stimulatory second signal is needed for full activation of cells, and without it they cannot function. Therefore, co-stimulation blockade has been proposed as a therapeutic modality for autoimmunity and transplantation. (Bluestone JA, St Clair EW, Turka LA. Immunity 2006; 24: 233-38.)

Naive T lymphocytes travel to T-cell areas of secondary lymphoid organs in search of antigen presented by antigen presenting cells (APC-s). Once activated, they proliferate vigorously, generating effector cells that can migrate to inflamed tissues to fight infection or in case of autoimmunity destroy tissues. Upon clearance of the antigen a fraction of primed/activated T lymphocytes persists as circulating memory cells that can normally confer protection and give, upon secondary challenge, an enhanced response. Two major types of memory T-cells remain: central memory cells, which patrol lymphoid organs, and effector memory cells that act as sentinels in peripheral tissues such as the skin and the gut.

Type 1 diabetes (T1DM) autoimmunity is driven by activated T-lymphocytes. Abatacept is a co-stimulation modulator and blocks full T-lymphocyte activation. The effect of two-year administration of abatacept in a randomized double-masked trial in recently diagnosed T1DM patients has been evaluated. Abatacept slowed decline of beta cell function significantly over two years. Preliminary results from this trial have been published as an article entitled "Co-stimulation modulation with abatacept in patients with recent-onset Type 1 diabetes: a randomized, double-blind, placebo-controlled trial" in The Lancet (published online June 28, 2011). This paper is included as Appendix A and is part of the presently filed application.

Multiple T-cell markers have been analyzed for any correlation to the progression of diabetes (destruction of remaining insulin-secreting β-cells). For patients treated with a compound that slows the autoimmune destruction in patients having diabetes humoral biomarker(s) (GAA, ICA512AA, IAA), it was found that the CD4 T-cell naive (CD45RO-CD62L+) to central memory (CD45RO+CD62L+) subpopulation ratios increased significantly from baseline during treatment and then returned to baseline after the therapy concluded. The treatment with abatacept was also found to significantly slow the decline of C-peptide by reducing the levels of CD4 T central memory (CD45RO+CD62L+) cells.

For patients not treated to slow the progression of diabetes (destruction of remaining insulin-secreting β-cells), it was found that higher central memory T-cells were significantly associated with subsequent decline in C-peptide. Thus, a decrease in these T-cells in the treated group was significantly associated with slower rate of C-peptide decline and this T immune cell subpopulation (central memory T-cells) can be used as a surrogate immune marker for self-insulin production decline.

Thus, it is hypothesized that abatacept blocks naive cells from becoming activated and the presence of a higher concentration of CD4 naive T-cells as compared to the CD4 memory T-cells indicates that the compound is effective at delaying the onset of T1DM in pre-diabetic subjects and is effective delaying the decline of insulin production in T1DM patients. Abatacept exert its effect on autoimmunity by reducing CD4 central memory T-cells levels as it blocks CD4 naive to CD4 central memory T-cell activation process This biomarker can also be used in the absence of a compound such as abatacept for the diagnosis of progressivness of diabetes or pre-diabetes (in conjunction with diabetes antibodies) as well as to determine the susceptibility to fast progressing diabetes mellitus. This marker can monitor the intensity and aggressiveness of autoimmune destruction, the speed of loss of the insulin-secreting β-cells.

The biomarker analysis as described herein may be provided in conjunction with known antibody testing. Such a combination provides both a determination of susceptibility to diabetes as well as a time frame for onset. Post-clinical onset, it can predict the time to the total loss of self-insulin production- time to "total diabetes".

C-peptide is a 31 amino acid peptide that acts as a structural connection in proinsulin. It is released into circulation along with insulin when the proinsulin is enzymatically cleaved. Thus, low to undetectable levels of C-peptide are found in T1DM while T2DM patients earlier in their disease often have higher than normal insulin/C-peptide level. However, there can be several reference ranges for C-peptide levels dependent upon factors such as the type of assay used, patient age, and whether or not a patient has fasted prior to the test. Any known assay method may be used to quantify C-peptide such as the radioimmuno assay (RIA) and immunochemiluminometric assay (ICMA). In the RIA method, C-peptide can be measured using goat anti-C-peptide. The antibody, which also recognizes proinsulin, has no cross-reactivity with insulin. The analytic sensitivity of the test is generally 0.125 ng/ml and an overnight fast is required. The RIA method provides a reference range for normal adults of 0.5 - 2 ng/mL. In the ICMA method, a competitive immunoassay having two incubation cycles is used to provide an analytic sensitivity of approximately 0.3 ng/mL. The ICMA method provides a reference range for normal adults of 0.9 - 4 ng/mL and the patient must be fasting. For children less than 12 years old, the reference range is 0.0 to 0.3 ng/mL. For children 10 - 12 years, the reference range is 0.4 to 3.3 ng/mL and for individuals 17 years and up. (LABCORP). In order to assess the capacity of the insulin-secreting β-cells to produce insulin food challenge tests are used, most commonly used test is called Mixed Meal Tolerance Test (MMTT) where several blood samples are collected over 2 or 4 hours time for C-peptide measurement.

As used herein, the term "subject" is a human or other animal, having or expected to have an autoimmune disorder. In embodiments of the invention "subjects" are human patients who have Type 1 diabetes mellitus. Thus, in embodiments the subject will be in need of the therapeutic treatment as provided herein. Preferred subjects are mammals. Examples of subjects include but at not limited to, humans, horses, monkeys, dogs, cats, mice, rats, cows, pigs, goats and sheep. In some embodiments, "subjects" are human patients who have been diagnosed with diabetes within the last 200, 100 or 50 days. In some embodiments, "subjects" are human patients who have been recently diagnosed with diabetes mellitus but still have residual beta-cell function.

A subject having Type 1 diabetes can be selected by evaluating subjects based on the diagnosis criteria for Type 1 diabetes. Alternatively, or in addition, this patient population can be selected by evaluating any genetic markers or autoantibodies or other biomarkers known to be correlated with Type 1 diabetes.

The term "treatment" or "treating" as used herein is defined as the application or administration of a therapeutic agent to a patient, or application or administration of a therapeutic agent to an isolated tissue or cell line from a patient, who has a disease, a symptom of disease or a predisposition toward a disease. Treatment is intended to encompass preventing the onset, slowing the progression, reversing or otherwise ameliorating, improve, or affect the disease, the symptoms or of disease or the predisposition toward disease. For example, treatment of a subject, e.g., a human subject, with a composition described herein, can slow, improve, or stop the ongoing autoimmunity, e.g., a reaction against pancreatic β-cells, in a subject before, during, or after the clinical onset of Type 1 diabetes.

The term a "diabetic condition" as used herein is intended to encompass diabetes, pre-diabetes, or a susceptibility to diabetes.

The treatment may be treatment using an approved pharmaceutical ingredient for clinical testing or may be the treatment occurring during a clinical trial or a pre-clinical trial.

The phrase "delaying the progression" as used herein in the context of delaying the progression of diabetes mellitus means that the loss of functional residual β-cell mass, before or after the clinical onset of Type 1 diabetes is delayed. The delay, for example, may be a delay of 1, 2, 3, 4, 5, 6, 9, 12, 15, 18, 21, 24 or more months, or it may be a delay of 2, 3, 4, or more years.

As used herein, the terms "administering" or "administration" are intended to encompass all means for directly and indirectly delivering a pharmaceutical composition to its intended site of action.

The term "therapeutically effective amount" refers to an amount effective, at dosages and for periods of time necessary, to achieve the desired therapeutic result. A therapeutically effective amount of a pharmaceutical composition may vary according to factors such as the disease state, age, sex, and weight of the individual, and the ability of the pharmaceutical composition elicit a desired response in the individual. A therapeutically effective amount is also one in which any toxic or detrimental effects of the pharmacological agent are outweighed by the therapeutically beneficial effects.

### EXAMPLES

Aspects of the applicant's teachings may be further understood in light of the following examples, which should not be construed as limiting the scope of the applicant's teachings in any way.

### Example 1 - Trial

As described in the above-referenced Lancet paper,
a phase 2 clinical trial was conducted of the use of abatacept for patients diagnosed with Type 1 diabetes. Eligible patients had been diagnosed with Type 1 diabetes within the past 100 days and had at least one diabetes-related autoantibody (microassayed insulin antibodies; glutamic acid decarboxylase-65 [GAD-65] antibodies; islet-cell antigen-512 [ICA-512] antibodies; or islet-cell autoantibodies) and had stimulated C-peptide concentrations of 0.2 nmol/L or higher.

Patients were randomly assigned in a 2:1 ratio, stratified by participating site, to receive experimental treatment with abatacept or placebo using a double blind protocol. Abatacept (Orencia, Bristol-Myers Squibb, Princeton, NJ, USA) was given on days 1, 14, and 28, and then every 28 days with the last dose on day 700 (total 27 doses) as a 30-min intravenous infusion at a dose of 10 mg/kg (maximum 1000 mg per dose) in a 100 mL 0.9% sodium chloride infusion. Normal saline infusion was used as placebo. Patients did not receive any premedication.

Blood samples were analyzed centrally. C-peptide concentrations were measured from frozen plasma with a two-site immunoenzymometric assay (Tosoh Bioscience, South San Francisco, CA, USA). Blood samples were obtained at 3, 6, 12, 18, and 24 months as well as at 30 months, six months after the end of the dosing.

Of the 112 patients enrolled in the study, 77 were randomly assigned to receive experimental treatment with abatacept and 35 were assigned to receive placebo. Results showed that over 2 years co-stimulation modulation with abatacept slows the reduction in β-cell function in recent-onset Type 1 diabetes by 9·6 months. At two year, the abatacept treated group had a 59% higher self-insulin production compare to placebo group. The abatacept treated group also had a significantly better HbAlc (measurement of level of blood sugar control) throughout the trial (with same insulin usage). The early intervention beneficial effect suggests that T-cell activation still occurs around the time of clinical diagnosis of Type 1 diabetes, even though the disease course has presumably been in progress for several years.

### Example 2 - Flow Cytometry

Flow cytometry analysis was performed on blood samples from subjects of the clinical trial in Example 1 for both abatacept and placebo arms at 0, 3, 6, 12, and 24 months with an additional analysis done six months after the end of the trial (30 months). Flow cytometry is a routine technique for counting and examining microscopic particles, such as cells, by suspending them in a stream of fluid and passing them one cell at the time by laser and an electronic detection apparatus. Modern instruments usually have multiple lasers and fluorescence detectors. Increasing the number of lasers and detectors allows for multiple antibody labeling, and can more precisely identify a target population by their phenotypic markers.

Fluorescence-activated cell sorting (FACS), a specialized type of flow cytometry, was used in the analysis. FACS provides a method for sorting a heterogeneous mixture of biological cells into two or more containers, one cell at a time, based upon the specific light scattering and fluorescent characteristics of each cell and characterizing them. It is a useful scientific instrument, as it provides fast, objective and quantitative recording of fluorescent signals from individual cells as well as physical separation of cells of particular interest. Fluorescent signal comes from the fluorescent labeled antibodies the cells have been incubated with prior the FACS. With multiple labeling, each antibody is coupled with a different fluorophore. Antibodies used are specific for the cell marker of interest. To detect CD4+ cells, antiCD4 antibody was labeled with a fluorophore. For the simultaneously detection of CD45RO, a specific antiCD45RO antibody with another fluorophore was also used. (Fluorescence-labeled antiCD4 and antiCD45RO antibodies are commercially available from various sources, such as BD Biosciences of San Jose, CA.) Each fluorophore has a characteristic peak excitation and emission wavelength, thus make it possible to distinguish between them, e.g., by using a fluorescence-activated cell sorting instruments, such as the Becton-Dickinson FACSCalibur or FACSAria system.

In three 5-color assays seven T-cell markers were studied. There were no changes from baseline found in the placebo group for any of these markers. In the treated group we saw no change in CD4 and CD8 T-cells or in naive and memory subsets of CD8 T-cells.

However, the CD4 T-cell naive (CD45RO-CD62L+) to central memory (CD45RO+CD62L+) subpopulation ratio increased significantly from baseline in the abatacept group during treatment and then returned to baseline off therapy. During the trial for the placebo group, higher CD4 central memory T-cells were significantly associated with subsequent decline in C-peptide. A decrease in these T-cells in abatacept group was significantly associated with slower rate of C-peptide decline.

The study also found that the regulatory T-cell population (CD4+CD25high) decreased from baseline in the abatacept group then returned to baseline off therapy. However, the reduction in these regulatory T-cells showed no correlation with the changes in naïve/memory populations or with changes in C-peptide levels.

Table 1 provides the least squares mean change from baseline, given as a log, of the ratio of CD4 naive T-cells to CD4 central memory T-cells and the standard deviation and p values for 3, 6, 12, and 24 months from baseline. The 30-months from baseline, where the subjects are off therapy is given as the 30-month data. The p values between drug and placebo groups are between groups at the same visit.

**TABLE 1**

| **LOG (NAÏVE T-CELLS/ CENTRAL MEMORY T-CELLS)** | | | | |
|---|---|---|---|---|
| | Month from baseline | Mean Change | Standard Error | p value |
| Abatacept | 3 | 2.137 | 0.1318 | p=NS |
| Placebo | 3 | 1.753 | 0.1927 | |
| Abatacept | 6 | 2.517 | 0.1305 | p=0.0002 |
| Placebo | 6 | 1.636 | 0.1949 | |
| Abatacept | 12 | 2.698 | 0.1305 | p=0.0002 |
| Placebo | 12 | 1.793 | 0.1951 | |
| Abatacept | 24 | 2.656 | 0.1328 | p=0.0001 |
| Placebo | 24 | 1.698 | 0.1954 | |
| Abatacept | 30 | 1.731 | 0.1323 | p=NS |
| Placebo | 30 | 1.623 | 0.2075 | |

The C-peptide concentration for these samples and time points were also measured. Analyzing both group data, prior changes both in CD4 T cell naive to central memory ratios and in CD4 central memory T cell levels predicted subsequent C-peptide loss in T1DM (FIG. 1) whereas contemporaneous T-cell measurements were not significantly associated with C-peptide change from baseline. It has been found that an increase in central memory T-cells is significantly associated with subsequent declines in C-peptide (self insulin production). Specifically, each unit of increase from baseline in Log central memory ratio is estimated to decrease C-peptide on average by -0.178 ng/ml. FIG. 1 provides the decrease of CD4 central memory T cell and increase in CD4 naïve/central memory T cell ratio resulting in reduction in C-peptide loss. The number of the unit change can be compared and quantitatively express providing a measure indicating the aggressiveness of the autoimmune processes and also the level of effectiveness of different intervention modalities. The level of effectiveness of different intervention modalities can then be ranked based on the number of units.

The time lags we looked at were 3 or 6 months. So for our purposes we need two different measurements of this cell population to assess the subsequent C-peptide loss or lack of it. Changes in these T-cell populations - both CD4 naïve/central memory cell ratios and CD4 central memory cell levels- predict subsequent C-peptide loss. FIGS. 2A - 2D shows the change in naive, memory and naive/memory T cell over time relative to baseline values. These show the changes as they occur over 30 months. While we looked at 3 and 6 months, as can be seen from FIG 2, other times and time intervals would work as well, and may include a baseline measurement taken prior to treatment, and/or measurements at 0, 1, 2, 3, 4, 5, 6, 7, 8, 9, 10, 11, 12, 15, 18, 21, 24, 27, 30, 33, 36, or more months.

The section headings used herein are for organizational purposes only and are not to be construed as limiting the subject matter described in any way. While the applicant's teachings are described in conjunction with various embodiments, it is not intended that the applicant's teachings be limited to such embodiments.

## Claims

1. A method of determining the effectiveness of a therapy to preserve β cell function in a subject who has Type 1 diabetes mellitus comprising:
selecting a subject who has Type 1 diabetes mellitus undergoing said therapy, and
(i) measuring the CD4 central memory (CD45RO+CD62L+) T-cell subpopulation in a sample from said subject, and evaluating the effectiveness of the therapy, wherein a low or decreasing CD4 central memory T-cell level during said therapy indicates effective therapy; or
(ii) measuring the CD4 central memory (CD45RO+CD62L+) T-cell subpopulation and measuring the CD4 T-cell naive (CD45RO-CD62L+) subpopulation in a sample from said subject, and evaluating the effectiveness of the therapy, wherein a high or increasing ratio of the CD4 T-cell naive to CD4 central memory T-cell subpopulation during said therapy indicates effective therapy.

2. The method of claim 1, further comprising:
determining the presence of a diabetes-related autoantibody.

3. The method of claim 1 or claim 2, wherein said sample is incubated with a labeled antiCD45RO antibody and a labeled antiCD62L antibody prior to the measuring step.

4. The method of any one of the preceding claims, wherein measuring comprises subjecting said sample to flow cytometry.

5. The method of any one of the preceding claims, wherein said sample is a blood sample.

6. The method of any one of the preceding claims, wherein the decreasing CD4 central memory T-cell level or the increasing ratio is relative to the level or ratio from said extracted sample at different time points.

7. The method of any one of claims 1-5, wherein the decreasing CD4 central memory T-cell level or the increasing ratio is relative to a standardized level or ratio, or to level or ratio obtained from a sample extracted before therapy starts.

8. The method of any one of the preceding claims, wherein a low or decreasing CD4 central memory T-cell level during said therapy indicates effective therapy.

9. The method of any one of the preceding claims, wherein measuring comprises measuring both the CD4 central memory T-cell subpopulation and the CD4 T-cell naive subpopulation and wherein a high or increasing ratio of the CD4 T-cell naive to CD4 central memory T-cell subpopulation during said therapy indicates effective therapy.

## Patentansprüche

1. Verfahren zum Bestimmen der Wirksamkeit einer Therapie, um eine β-Zellfunktion in einem Subjekt zu bewahren, das Typ 1 Diabetes mellitus aufweist, umfassend:
Auswählen eines Subjekts, das Typ 1 Diabetes mellitus aufweist und sich der Therapie unterzieht, und
(i) Messen der CD4-Zentralgedächtnis-, (CD45RO+CD62L+), T-Zell-Subpopulation in einer Probe aus dem Subjekt, und Bewerten der Wirksamkeit der Therapie, wobei ein niedriger oder abnehmender CD4-Zentralgedächtnis-T-Zellspiegel während der Therapie auf eine wirksame Therapie hindeutet; oder
(ii) Messen der CD4-Zentralgedächtnis-, (CD45RO+CD62L+), T-Zell-Subpopulation und Messen der naiven CD4-T-Zell-, (CD45RO-CD62L+), Subpopulation in einer Probe aus dem Subjekt, und Bewerten der Wirksamkeit der Therapie, wobei ein hohes oder zunehmendes Verhältnis der naiven CD4-T-Zell- zur CD4-Zentralgedächtnis-T-Zell-Subpopulation während der Therapie auf eine wirksame Therapie hindeutet.

2. Verfahren nach Anspruch 1, weiter umfassend:
Bestimmen des Vorhandenseins eines diabetesbedingten Autoantikörpers.

3. Verfahren nach Anspruch 1 oder Anspruch 2, wobei die Probe mit einem markierten Anti-CD45RO-Antikörper und einem markierten Anti-CD62L-Antikörper vor dem Messschritt inkubiert wird.

4. Verfahren nach einem der vorstehenden Ansprüche, wobei das Messen ein Unterwerfen der Probe einer Durchflusszytometrie umfasst.

5. Verfahren nach einem der vorstehenden Ansprüche, wobei die Probe eine Blutprobe ist.

6. Verfahren nach einem der vorstehenden Ansprüche, wobei der abnehmende CD4-Zentralgedächtnis-T-Zellspiegel oder das zunehmende Verhältnis relativ zum Spiegel oder Verhältnis aus der entnommenen Probe an verschiedenen Zeitpunkten ist.

7. Verfahren nach einem der Ansprüche 1-5, wobei der abnehmende CD4-Zentralgedächtnis-T-Zellspiegel oder das zunehmende Verhältnis relativ zu einem standardisierten Spiegel oder Verhältnis, oder zu einem Spiegel oder Verhältnis ist, der/das aus einer vor Therapiebeginn entnommenen Probe erhalten wird.

8. Verfahren nach einem der vorstehenden Ansprüche, wobei ein niedriger oder abnehmender CD4-Zentralgedächtnis-T-Zellspiegel während der Therapie auf eine wirksame Therapie hindeutet.

9. Verfahren nach einem der vorstehenden Ansprüche, wobei das Messen sowohl ein Messen der CD4-Zentralgedächtnis-T-Zell-Subpopulation als auch der naiven CD4-T-Zell-Subpopulation umfasst und wobei ein hohes oder zunehmendes Verhältnis der naiven CD4-T-Zell- zur CD4-Zentralgedächtnis-T-Zell-Subpopulation während der Therapie auf eine wirksame Therapie hindeutet.

## Revendications

1. Procédé de détermination de l'efficacité d'une thérapie pour préserver la fonction des cellules β chez un sujet qui a un diabète sucré de type 1 comprenant :
la sélection d'un sujet qui a un diabète sucré de type 1 subissant ladite thérapie, et
(i) la mesure de la sous-population de lymphocytes T de mémoire centrale CD4 (CD45RO+CD62L+) dans un échantillon issu dudit sujet et l'évaluation de l'efficacité de la thérapie, dans lequel un niveau de lymphocytes T faible ou décroissant de la mémoire centrale CD4 au cours de ladite thérapie indique une thérapie efficace ; ou
(ii) la mesure de la sous-population de lymphocytes T de mémoire centrale CD4 (CD45RO+CD62L+) et la mesure de la sous-population de lymphocytes T naïfs de CD4 (CD45RO-CD62L+) dans un échantillon issu dudit sujet, et l'évaluation de l'efficacité de la thérapie, dans lequel un rapport élevé ou croissant de lymphocytes T naïfs de CD4 à la sous-population de lymphocytes T de mémoire centrale CD4 au cours de ladite thérapie indique une thérapie efficace.

2. Procédé selon la revendication 1, comprenant en outre :
la détermination de la présence d'un auto-anticorps rapporté au diabète.

3. Procédé selon la revendication 1 ou la revendication 2, dans lequel ledit échantillon est incubé avec un anticorps anti-CD45RO marqué et un anticorps antiCD62L marqué avant l'étape de mesure.

4. Procédé selon l'une quelconque des revendications précédentes, dans lequel la mesure comprend la soumission dudit échantillon à une cytométrie en flux.

5. Procédé selon l'une quelconque des revendications précédentes, dans lequel ledit échantillon est un échantillon de sang.

6. Procédé selon l'une quelconque des revendications précédentes, dans lequel le niveau décroissant de lymphocytes T de mémoire centrale CD4 ou le rapport croissant est en rapport avec le niveau ou le rapport desdits échantillons extraits à différents moments.

7. Procédé selon l'une quelconque des revendications 1 à 5, dans lequel le niveau décroissant de lymphocytes T de mémoire centrale CD4 ou le rapport croissant est relatif à un niveau ou à un rapport standardisé ou à un niveau ou à un rapport obtenu à partir d'un échantillon extrait avant de démarrer la thérapie.

8. Procédé selon l'une quelconque des revendications précédentes, dans lequel un niveau faible ou décroissant de lymphocytes T de mémoire centrale CD4 au cours de ladite thérapie indique une thérapie efficace.

9. Procédé selon l'une quelconque des revendications précédentes, dans lequel la mesure comprend la mesure à la fois de la sous-population de lymphocytes T de mémoire centrale CD4 et de la sous-population de lymphocytes T naïfs de CD4 et dans lequel un rapport élevé ou croissant des lymphocytes T naïfs de CD4 à la sous-population de lymphocytes T de mémoire centrale CD4 au cours de ladite thérapie indique une thérapie efficace.
